# EUROPÄISCHE PATENTSCHRIFT

(11) **EP 1 733 687 B1**
(45) Veröffentlichungstag und Bekanntmachung des Hinweises auf die Patenterteilung: **02.12.2009**
(21) Anmeldenummer: 06011989.8
(22) Anmeldetag: 10.06.2006
(51) Int. Cl.: A61B 17/32, A61B 17/28

(54) **Medizinisches Greif- und/oder Schneidinstrument**
Medical gripping and/or cutting instrument
Instrument medical d'agrippement et/ou de coupe

(30) Priorität: 14.06.2005 DE 102005027418
(43) Veröffentlichungstag der Anmeldung: 20.12.2006
(73) Patentinhaber: University of Dundee, Dundee DD1 4HN (GB)
(72) Erfinder: Cuschieri, Alfred, St. Andrews Fife KY16 9TY (GB); Frank, Timothy Graham, Newport-On-Tay Fife KY16 9TY (GB); Brown, Stuart I., St. Andrews Fife KY16 8QX (GB)
(74) Vertreter: Hofmeister, Frank

(56) Entgegenhaltungen:
- EP-A- 0 827 715
- WO-A-2005/112795
- DE-A1- 4 428 479
- US-A- 5 234 460
- US-A- 5 542 843
- US-A- 5 908 436
- ANONYMOUS: "FELCO Pruners" INTERNET ARTICLE, [Online] 6. Dezember 2003 (2003-12-06), XP002400954 Gefunden im Internet: URL:http://web.archive.org/web/20031206093 441/http://www.felcostore.com/pruners.jsp> [gefunden am 2006-09-21]

## Beschreibung

Die Erfindung betrifft ein medizinisches Greif- und/oder Schneidinstrument mit einem Schaft, an dessen distalem Ende ein aus mindestens zwei Maulteilen bestehendes Werkzeug angeordnet ist und an dessen proximalem Ende eine aus mindestens zwei Griffteilen bestehende Handhabe angeordnet ist, wobei mindestens ein Maulteil des Werkzeugs zum Öffnen und Schließen über ein verschwenkbar ausgebildetes Griffteil der Handhabe gegenüber dem anderen Maulteil des Werkzeugs verstellbar ist.

Gattungsgemäße medizinische Instrumente sind in verschiedenen Ausführungsformen bekannt. Da ein mittels der Maulteile des Werkzeugs ergriffenes Teil, beispielsweise eine chirurgische Nadel, oft über einen längeren Zeitraum fest zwischen den Maulteilen des Werkzeugs fixiert werden muss, muss der Operateur die beiden Griffteile der Handhabe fortwährend zusammengepresst halten. Bei längerem Arbeiten können die Griffteile der Handhabe so stark an der Handinnenfläche des Benutzers reiben, dass Blasen entstehen. Neben dieser möglichen Blasenbildung ist bei den bekannten medizinischen Instrumenten nachteilig, dass die Griffhaltung nicht immer die bestmögliche Krafteinleitung über die Hand auf das Instrument gewährleistet.

Ein gattungsgemäßes Instrument ist aus EP 0 827 715 A2 bekannt auf der der Oberbegriff des Anspruchs 1 basiert.

Davon ausgehend liegt der Erfindung die A u f g a b e zugrunde, ein medizinisches Greif- und/oder Schneidinstrument zu schaffen, das ein handschonendes Arbeiten bei gleichzeitig optimierter Bedienung ermöglicht.

Die L ö s u n g dieser Aufgabenstellung ist erfindungsgemäß dadurch gekennzeichnet, dass zumindest ein Teilbereich mindestens eines Griffteils der Handhabe um die Längsachse dieses Griffteils verschwenkbar ist.

Durch die erfindungsgemäße Ausbildung eines Teilbereichs eines Handgriffs als um die Längsachse des Griffteils verschwenkbarer Teilbereich ist es erstmalig möglich, ohne Loslassen oder Umgreifen der Handhabe die Lage der Handhabe in der Hand einerseits so zu variieren, dass keine Druckstellen an der Hand des Operateurs entstehen und andererseits eine stets optimale Lage der Griffteile zur Hand- und Fingerstellung des Operateurs gewährleistet ist, um die bestmögliche Krafteinleitung über die Hand auf das Instrument zu ermöglichen.

Gemäß einer praktischen Ausführungsform der Erfindung wird vorgeschlagen, dass ein Griffteil der Handhabe aus einem starren Teilbereich sowie einem verschwenkbaren Teilbereich bestehend zweiteilig so aufgebaut ist, dass der verschwenkbare Teilbereich zumindest teilweise um den starren Teilbereich verschwenkbar ist. Als alternative zur Ausbildung eines Griffteils als im Ganzen um seine Längsachse verdrehbar ist diese Zweiteilung des Griffteils vorteilhaft, da der starre Teilbereich der Konstruktion dem Instrument die für die gezielte Handhabung notwendige Stabilität verleiht.

Um den verschwenkbaren Teilbereich des Griffteils sicher ergreifen zu können und das Verschwenken zu erleichtern, wird erfindungsgemäß vorgeschlagen, dass an dem verschwenkbaren Teilbereich des Griffteils Griffmulden ausgebildet sind.

Weiterhin wird mit der Erfindung vorgeschlagen, dass der Verschwenkwinkel des verschwenkbaren Teilbereichs über Anschläge begrenzbar ist, um ein Verrutschen des Griffteils in der Hand des Operateurs zu verhindern.

Schließlich wird mit der Erfindung vorgeschlagen, dass die Griffteile der Handhabe über ein Federelement in die geöffnete Stellung vorgespannt sind. Diese Vorspannung der Griffteile dient dazu, die Griffteile der Handhabe ohne Zutun des Operateurs in die geöffnete Stellung zu überführen.

Weitere Merkmale und Vorteile der Erfindung ergeben sich anhand der zugehörigen Zeichnung, in der ein Ausführungsbeispiel eines erfindungsgemäßen medizinischen Greif- und/oder Schneidinstruments nur beispielhaft dargestellt ist. In der Zeichnung zeigt:
- Fig. 1: eine Seitenansicht eines erfindungsgemäßen medizinisches Greif-und/oder Schneidinstruments in einer ersten Arbeitsstellung der Griffteile;
- Fig. 2: eine Rückansicht der Darstellung gemäß Fig. 1;
- Fig. 3: eine Seitenansicht des medizinischen Greif- und/oder Schneidinstruments gemäß Fig. 2, jedoch die Griffteile in einer zweiten Arbeitsstellung darstellend und
- Fig. 4: eine Rückansicht der Darstellung gemäß Fig. 3.

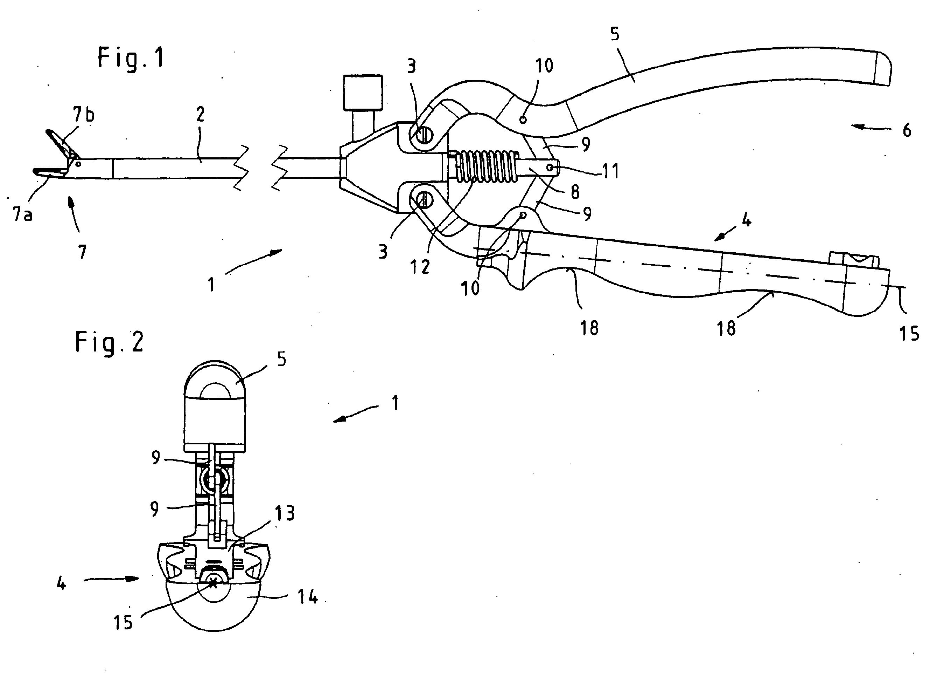

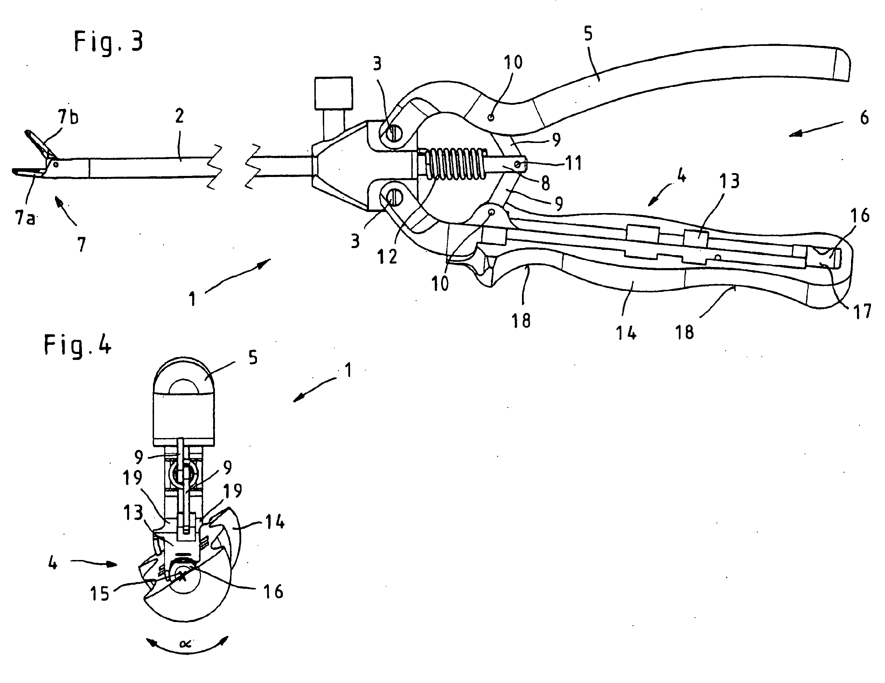

Die Abbildungen Fig. 1 und 3 zeigen geschnittene Seitenansichten eines medizinischen Greif- und/oder Schneidinstruments 1, dessen Kraftübertragungsmechanismus vielseitig verwendet werden kann, wie beispielsweise für Stanzen, Scheren, Nadelhalter, Fassinstrumente und dergleichen.

Das dargestellte medizinische Instrument 1 besteht im Wesentlichen aus einem hohlen Schaft 2, an dessen proximalem Ende eine aus zwei, um jeweils zugehörige Schwenkachsen 3 verschwenkbaren Griffteilen 4 und 5 bestehende Handhabe 6 angeordnet ist. Am distalen Ende des Schaftes 2 ist ein Werkzeug 7 angeordnet, welches beim dargestellten Ausführungsbeispiel aus einem starr mit dem Schaft 2 verbundenen Maulteil 7a sowie einem verschwenkbaren Maulteil 7b besteht. Zum Öffnen und Schließen der Maulteile 7a und 7b des Werkzeugs 7 über die Betätigung der verschwenkbaren Griffteile 4 und 5 der Handhabe 6 stehen die Griffteile 4, 5 und das verschwenkbare Maulteil 7b über eine Zug-/Druckstange 8 miteinander in Wirkverbindung.

Wie aus den Abbildungen Fig. 1 und 3 ersichtlich, erfolgt die Kopplung der Griffteile 4 und 5 mit der Zug-/Druckstange 8 bei der dargestellten Ausführungsform über zwischengeschaltete Schwenkhebel 9, die mit einem freien Ende verschwenkbar an einem Lagerpunkt 10 an einem der Griffteile 4, 5 und mit dem anderen freien Ende an einer gemeinsamen Schwenkachse 11 an der Zug-/Druckstange 8 gelagert sind. Die Anlenkung der Griffteile 4 und 5 über die Schwenkhebel 9 an der Zug-/Druckstange 8 ist derart ausgelegt, dass beim Zusammendrücken der Griffteile 4, 5 die Zug-/Druckstange 8 über die Schwenkhebel 9 zum proximalen Ende des medizinischen Instruments 1 gezogen wird, was wiederum ein Verschwenken des verschwenkbaren Maulteils 7b des Werkzeugs 7 in die geschlossene Stellung bewirkt.

Zum Öffnen der Maulteile 7a, 7b des Werkzeugs 7 bedarf es somit des Auseinanderbewegens der Griffteile 4, 5 der Handhabe 6, wodurch die Zug-/Druckstange 8 über die Schwenkhebel 9 zum distalen Ende des medizinischen Instruments 1 gedrückt wird. Um das Auseinanderdrücken der Griffteile 4, 5 der Handhabe 6 zu erleichtern, ist auf der Zug-/Druckstange 8 ein als Zugfeder ausgebildetes Federelement 12 angeordnet, über das die Griffteile 4, 5 in die geöffnete Stellung vorgespannt sind.

Um dem Operateur zu ermöglichen, auch ohne Loslassen oder neues Ergreifen der Griffteile 4 und 5 der Handhabe 6 die Lage der Griffteile 4 und 5 in der Hand zu verändern, ist der Griffteil 4 zweiteilig aufgebaut, nämlich bestehend aus einem starren Teilbereich 13 sowie einem relativ zum starren Teilbereich 13 verschwenkbaren Teilbereich 14, wie dies insbesondere der Abbildung Fig. 4 zu entnehmen ist. Bei der dargestellten Ausführungsform ist der verstellbare Teilbereich 14 um die Längsachse 15 des Griffteils 4 verschwenkbar an dem starren Teilbereich 13 des Griffteils 4 gelagert, wobei die gegenseitige Lagerung der Teilbereiche 13 und 14 aneinander über am starren Teilbereich 13 angeformte Lagerzapfen 16 erfolgt, die in Lageraufnahmen 17 am verschwenkbaren Teilbereich 14 gelagert sind.

Wie aus den Abbildungen Fig. 1 und 3 ersichtlich, sind am verschwenkbaren Teilbereich 14 des Griffteils 4 Griffmulden 18 zur Aufnahme der Finger der Haltehand ausgeformt, wodurch das Ergreifen und Verschwenken des verschwenkbaren Teilbereichs 14 erleichtert wird.

Alternativ zu der dargestellten Ausführungsform ist es selbstverständlich auch möglich, einen Griffteil 4 oder 5 so auszugestalten, dass dieser abwechselnd in starre Teilbereiche 13 und verschwenkbare Teilbereiche 14 unterteilt ist, also beispielsweise der Bereich mit der proximalen Griffmulde 18 starr ausgebildet ist, während der die distale Griffmulde 18 aufweisende Bereich verschwenkbar ausgestaltet ist.

Gemäß einer weiteren konstruktiven Ausgestaltungsform besteht die Möglichkeit, den gesamten Griffteil 4 oder 5 einer Handhabe 6 eines medizinischen Instruments 1 als um seine Längsachse 15 verdrehbar auszugestalten.

Die Handhabung des zuvor beschriebenen und in den Abbildungen Fig. 1 und 2 dargestellten medizinischen Instruments 1 geschieht wie folgt:

In der in Fig. 1 und 2 dargestellten Ausgangsstellung, in der die Griffteile 4 und 5 der Handhabe 6 im Wesentlichen horizontal und parallel zueinander ausgerichtet sind, wie dies die Rückansicht gemäß Fig. 2 verdeutlicht, ergreift der Operateur das medizinische Instrument 1, um beispielsweise eine chirurgische Nadel mittels des Werkzeugs 7 zu ergreifen und zu halten. Hierzu drückt der Operateur die mit einer Hand ergriffenen Griffteile 4 und 5 der Handhabe 6 zusammen, bis die Maulteile 7a und 7b geschlossen sind.

Da die Griffteile 4 und 5 über das Federelement 12 in die Offenstellung vorgespannt sind, muss der Operateur die Griffteile 4 und 5 fest ergriffen halten, wenn der Halt des Werkzeugs 7 nicht gelockert werden soll. Bei längerem Arbeiten üben die Griffteile 4 und 5 der Handhabe 6 dabei punktuell einen stetigen Druck auf die Handinnenfläche des Benutzers aus, der zu Ermüdung und sogar Blasenbildung führen kann.

Um die Lage der Griffteile 4 und 5 in seiner Hand zu verändern, ohne aber die Handhabe 6 loslassen zu müssen, kann der Operateur den mit den Fingern der Haltehand umgriffenen verschwenkbaren Teilbereich 14 des Griffteils 4 um die Längsachse 15 des Griffteils 4 verschwenken, wie dies in Fig. 3 und 4 dargestellt ist, wodurch sich auch die Lage des Griffteils 5 an der Handinnenfläche des Operateurs ändert und so zu einer Entlastung der Haltehand führt.

Neben der Entlastung der Haltehand bewirkt die Verschwenkbarkeit zumindest eines Teilbereichs 14 des Griffteils 4, dass der Operateur bei jeder Handstellung die Griffteile 4 und 5 der Handhabe 6 immer so zueinander einstellen kann, dass eine bestmögliche Krafteinleitung über die Haltehand auf das medizinische Instrument 1 gewährleistet ist.

Der Verschwenkwinkel α des verschwenkbaren Griffteils 14 kann über Anschläge 19 begrenzt werden, um ein Überdrehen oder Verrutschen des Griffteils 4 in der Haltehand zu verhindern.

Ein solchermaßen ausgebildetes medizinisches Instrument 1 zeichnet sich dadurch aus, dass der Operateur zur Entlastung der Haltehand den Halt der Griffteile 4 und 5 der Handhabe 6 ohne Loslassen der Griffteile 4 und 5 ändern kann.

### B e z u g s z e i c h e n l i s t e

- 1: medizinisches Greif- und/oder Schneidinstrument
- 2: Schaft
- 3: Schwenkachse
- 4: Griffteil
- 5: Griffteil
- 6: Handhabe
- 7: Werkzeug
- 7a: starres Maulteil
- 7b: verschwenkbares Maulteil
- 8: Zug-/Druckstange
- 9: Schwenkhebel
- 10: Lagerpunkt
- 11: Schwenkachse
- 12: Federelement
- 13: starrer Teilbereich
- 14: verschwenkbarer Teilbereich
- 15: Längsachse
- 16: Lagerzapfen
- 17: Lageraufnahme
- 18: Griffmulde
- 19: Anschlag

- α: Verschwenkwinkel

## Patentansprüche

1. Medizinisches Greif- und/oder Schneidinstrument mit einem Schaft (2), an dessen distalem Ende ein aus mindestens zwei Maulteilen (7a, 7b) bestehendes Werkzeug (7) angeordnet ist und an dessen proximalem Ende eine aus mindestens zwei Griffteilen (4, 5) bestehende Handhabe (6) angeordnet ist, wobei mindestens ein Maulteil (7b) des Werkzeugs (7) zum Öffnen und Schließen über ein verschwenkbar ausgebildetes Griffteil (4, 5) der Handhabe (6) gegenüber dem anderen Maulteil (7a) des Werkzeugs (7) verstellbar ist und wobei mindestens ein Griffteil (4) der Handhabe (6) aus einem starren Teilbereich (13) sowie einem verschwenkbaren Teilbereich (14) bestehend zweiteilig so aufgebaut ist, dass der verschwenkbare Teilbereich (14) zumindest teilweise um den starren Teilbereich (13) verschwenkbar ist,
**dadurch gekennzeichnet,**
**dass** die Griffteile (4, 5) der Handhabe Längsachsen aufweisen, die (6) in Richtung der Instrumentenlängsachse ausgerichtet sind, wobei der verschwenkbare Teilbereich (14) des Griffteils (4) der Handhabe (6) so ausgebildet ist, dass er achsparallel um die Längsachse (15) dieses Griffteils (4) verschwenkbar ist, so dass sich die Lage des Griffteils an der Handinnenfläche des Operateurs ändern kann zur Entlastung der Haltehand.

2. Medizinisches Greif- und/oder Schneidinstrument nach Anspruch 1, **dadurch gekennzeichnet, dass** an dem verschwenkbaren Teilbereich (14) des Griffteils (4) Griffmulden (18) ausgebildet sind.

3. Medizinisches Greif- und/oder Schneidinstrument nach Anspruch 1 oder 2, **dadurch gekennzeichnet, dass** der Verschwenkwinkel (a) des verschwenkbaren Teilbereichs (14) über Anschläge (19) begrenzbar ist.

4. Medizinisches Greif- und/oder Schneidinstrument nach einem der Ansprüche 1 bis 3, **dadurch gekennzeichnet, dass** die Griffteile (4, 5) der Handhabe (6) über ein Federelement (12) in die geöffnete Stellung vorgespannt sind.

## Claims

1. Medical gripping and/or cutting instrument with a shaft (2), at the distal end of which a tool (7) is arranged comprising at least two jaw parts (7a, 7b) and at the proximal end of which a handle (6) is arranged comprising at least two grip parts (4, 5), with, for opening and closing, at least one jaw part (7b) of the tool (7) being adjustable with respect to the other jaw part (7a) of the tool (7) by means of a grip part (4, 5) of the handle (6) designed in a pivotable fashion and with at least one grip part (4) of the handle (6) being of a two-part design comprising a stiff portion (13) and a pivotable portion (14) such that the pivotable portion (14) can be at least partly pivoted about the stiff portion (13),
**characterized**
**in that** the grip parts (4, 5) of the handle (6) have longitudinal axes which are aligned in the direction of the longitudinal axis of the instrument, with the pivotable portion (14) of the grip part (4) of the handle (6) being designed such that it can be pivoted axially parallel about the longitudinal axis (15) of this grip part (4) and so the position of the grip part on the inner surface of the hand of the operator can be varied in order to relieve the holding hand.

2. Medical gripping and/or cutting instrument according to Claim 1, **characterized in that** recessed grips (18) are formed on the pivotable portion (14) of the grip part (4)

3. Medical gripping and/or cutting instrument according to Claim 1 or 2, **characterized in that** the pivot angle (α) of the pivotable portion (14) can be restricted by stops (19).

4. Medical gripping and/or cutting instrument according to one of Claims 1 to 3, **characterized in that** the grip parts (4, 5) of the handle (6) are prestressed in the opened position by means of a spring element (12).

## Revendications

1. Instrument médical de préhension et/ou de coupe comprenant un corps allongé (2) à l'extrémité distale duquel est agencé un outil (7) constitué d'au moins deux pièces de mâchoire (7a, 7b) et à l'extrémité proximale duquel est agencée une poignée de manoeuvre (6) constituée d'au moins deux pièces de poignée (4, 5), au moins une pièce de mâchoire (7b) de l'outil (7) pouvant être déplacée par rapport à l'autre pièce de mâchoire (7a) de l'outil (7) pour l'ouverture ou la fermeture, par l'intermédiaire d'une pièce de poignée (4, 5) de configuration pivotante de la poignée de manoeuvre (6), et au moins une pièce de poignée (4) de la poignée de manoeuvre (6), constituée d'un tronçon partiel fixe (13) ainsi que d'un tronçon partiel pivotant (14), étant conçue en deux parties de façon telle que le tronçon partiel pivotant (14) puisse pivoter au moins partiellement autour du tronçon partiel fixe (13),
**caractérisé**
**en ce que** les pièces de poignée (4, 5) de la poignée de manoeuvre (6) présentent des axes longitudinaux, qui sont orientés dans la direction de l'axe longitudinal de l'instrument, le tronçon partiel pivotant (14) de la pièce de poignée (4) de la poignée de manoeuvre (6) étant réalisé de façon à pouvoir pivoter parallèlement à l'axe autour de l'axe longitudinal (15) de cette pièce de poignée (4), de sorte qu'il est possible de faire varier la position de la pièce de poignée sur la surface interne de la main de l'opérateur, en vue de décharger ou de soulager la main assurant la tenue.

2. Instrument médical de préhension et/ou de coupe selon la revendication 1, **caractérisé en ce que** sur le tronçon partiel pivotant (14) de la pièce de poignée (4) sont réalisées des cavités ou empreintes de préhension (18).

3. Instrument médical de préhension et/ou de coupe selon la revendication 1 ou la revendication 2, **caractérisé en ce que** l'angle de pivotement (α) du tronçon partiel pivotant (14) peut être limité par des butées (19).

4. Instrument médical de préhension et/ou de coupe selon l'une des revendications 1 à 3, **caractérisé en ce que** les pièces de poignée (4, 5) de la poignée de manoeuvre (6) sont précontraintes dans la position ouverte, par l'intermédiaire d'un élément de ressort (12).
